# EUROPÄISCHE PATENTSCHRIFT

(11) **EP 1 561 486 B1**
(45) Veröffentlichungstag und Bekanntmachung des Hinweises auf die Patenterteilung: **05.12.2007**
(21) Anmeldenummer: 04012726.8
(22) Anmeldetag: 28.05.2004
(51) Int. Cl.: A61M 16/04

(54) **Vorrichtung zur Unterfütterung, Ummantelung und Lagesicherung von Trachealkanülen**
Device for surrounding, lining and holding tracheal tubes
Appareil pour entourer,revêtir et retenir en position des tubes trachéaux

(30) Priorität: 09.02.2004 DE 102004006361
(43) Veröffentlichungstag der Anmeldung: 10.08.2005
(73) Patentinhaber: MedicoMediTec Gesellschaft zum Vertrieb medizinischer Hilfsmittel mbH, 51519 Odenthal (DE)
(72) Erfinder:
(74) Vertreter: Cohausz & Florack

(56) Entgegenhaltungen:
- DE-A1- 19 932 697
- DE-U1- 20 310 956
- DE-U1- 29 710 855
- DE-U1- 29 911 972

## Beschreibung

Die Erfindung betrifft eine Vorrichtung zur Unterfütterung, Ummantelung und Lagesicherung von Trachealkanülen mit einer dem Körper zugewandten Deckschicht, einer zu der Kleidung weisenden Oberflächenschicht und einem zwischen Deckschicht und Oberflächenschicht befindlichen ein- oder mehrschichtigen Kern, mit einer alle Schichten durchbrechenden, im wesentlichen runden Öffnung zur Aufnahme der Trachealkanüle, wobei zwischen der Öffnung und dem äußeren Rand eine Durchtrennung vorgesehen sein kann.

In der HNO-Heilkunde kennt man derartige Vorrichtungen zur Unterfütterung, Ummantelung und Lagesicherung von Trachealkanülen schon seit vielen Jahren. Sie sind im Handel unter der Bezeichnung 'Tracheal-Kompressen' bekannt. Sie sollen den Patienten mit einem Tracheostoma, d.h. Menschen mit einem Luftröhrenschnitt oder Menschen mit dem in die Halsgrube operativ eingepflanzten oberen Luftröhrenende, einerseits die bei Zustand nach solchen Operationen erforderlichen Trachealkanülen unterfüttern und polstern, andererseits aber auch zum Aufsaugen des in vielen Fällen in großen Mengen anfallenden Trachealsekrets dienen.

Eine der ersten Tracheal-Kompressen-Ausführungen ist eine einseitige alubedampfte Wundkompresse, die erst durch nachträgliches Einstanzen einer kreisrunden, glattrandigen Öffnung zentral in das obere Kompressendrittel, zum Zwecke der Aufnahme der Trachealkanüle, zu einer Tracheal-Kompresse aufgearbeitet worden ist.

Im Laufe der folgenden Jahre fanden ständig Weiterentwicklungen an den Tracheal-Kompressen statt. Schon Anfang der 90er Jahre waren, auf den Anmelder zurückgehend, die sogenannten kammersystemischen Kompressen auf dem Markt (G 90 04 962.4). Sowohl bei den Kammer-Tracheal-Kompressen im Original als auch bei späteren, recht und schlecht gelungenen Nachahmungen finden Nonwoven-Vliesstoffe als Flüssigkeitsabsorbentes Anwendung. Hier handelt es sich vorwiegend um Krempel-, Spinn- oder nassgelegte Vliesstoffe.

Neben der am meisten gehandelten, sogenannten Zwei-Kammer-Kompresse werden nun auch Ein-Kammer-(DE 299 11 972 U1) und Drei-Kammer-Trachealkompressen (DE 297 10 855 U1) angeboten und gehandelt.

Es gibt die Tracheal-Kompressen in geschlossen-gelochter und in geschlitzt-gelochter Ausführung (DE 199 32 697 A1, DE 15 16 467 A1, DE 299 11 972 U1). Erstere können nur gegen eine frische ausgetauscht werden, wenn auch die Kanüle aus dem Stoma entfernt wird. Letzteres ist besonders postoperativ, aber in vielen Fällen auch noch später, unmöglich bzw. sogar verboten.

Für diese Fälle hat man die geschlitzt-gelochten Kompressenvarianten entwickelt. Hier kann die Trachealkanüle beim Wechsel der Tracheal-Kompresse im Stoma liegen bleiben und die neue Tracheal-Kompresse um das Kanülenrohr herum angelegt werden.

Mit Blick auf das sehr sensible Einsatzgebiet der Trachealkompressen, nämlich die Region um den ungehindert offen stehenden Zugang in das Bronchialorgan herum, ist von großem Nachteil, dass die heutigen Trachealkompressen einen starken Faser- und Faserstaubflug aufweisen. Diese rühren schon mal daher, dass die schmalen Vliesrollen zunächst von großen Mutterrollen abgeschnitten werden müssen. Dabei wird Druck auf das Material ausgeübt, und so setzen sich dann Staub und Schnittfasern an, auf und in den Schmalrollen ab. Dieser Prozess wiederholt sich beim weiteren Herausstanzen der Trachealkompressen aus den Schmalrollen und beim Einbringen der Durchbrüche und Durchtrennungen, so dass die Anwendung von Trachealkompressen solchen Materials und solcher Fabrikation immer gefahrbringend für die Atemwege ist.

Die Stanz- und Schnittkanten, welche an der gesamten Kompressenperipherie und an den Durchbrüchen bzw. Durchtrennungen entstehen, sind nicht nur verantwortlich für den mitunter sogar sehr starken Faser- und Staubflug, sondern sie sind auch die Schwachstellen für das Flüssigkeitsrückhaltevermögen der Trachealkompressen. Sie sind immer eine Leckage für absorbiertes Sekret.

Nachteilig an allen marktüblichen Typen der Trachealkompressen ist der hohe Herstellungspreis. Da es sich bei den in diesem Zusammenhang vorwiegend verwendeten Karden- oder Extrusions-Nonwovens-Vliesstoffen unter anderem um hochwertige Fasern Polyester, Polypropylen und Polyamid handelt, sind bereits die Materialkosten für die Rollenware empfindlich hoch. Der Preis des dann schließlich und in mehreren Schritten hergestellten Endproduktes tangiert immer mehr die Grenze der Wettbewerbsfähigkeit. Auf den Preis der heutigen Tracheal-Kompressen beispielsweise aus Kardenoder Spinn-Nonwovens schlägt sich auch die Tatsache, dass es diese Vliese bislang nur als Rollenware gibt, deren Lagerung, Transport und Weiterverarbeitung sich entsprechend umständlich und unwirtschaftlich gestalten.

Ein weiterer wesentlicher Nachteil der bekannten und auf dem Markt erhältlichen Tracheal-Kompressen besteht darin, dass es diese Vliesstoffe zwar in verschiedenen, wenn auch begrenzten, Dicken mit entsprechendem Volumen und ebenfalls entsprechend begrenzter WasseraufnahmeKapazität gibt, sie aber alle aus nur einer Lage, mit ein- oder beidseitiger Abdeckung, bestehen. Das bedeutet, werden eine höhere Absorptionskapazität und ein höherer Quellwert benötigt, muss entweder ein dickleibigerer und somit wesentlich teurer Vliesstoff beschafft werden oder es müssen, wie es bereits schon bei den Zwei- und Drei-Kammer-Tracheal-Kompressen geschieht, zwei oder drei Bahnen aufeinander befestigt werden, was ebenfalls den Endpreis entsprechend in die Höhe treibt.

Nachteilig ist des weiteren an den aktuellen Tracheal-Kompressen, dass der einlagige Vliesstoff zwar mitunter eine schnelle Absorption an sich hat und auch über eine gute Absorptionskapazität verfügt, aber keine geregelte Flüssigkeitsaufnahme, -verteilung, -weitergabe und -speicherung aufweist. Das bedeutet einen häufigeren Wechsel dieser Einwegprodukte (disposables), und somit auch mehr Kosten.

Schließlich seien als Nachteile die eingeschränkten Rücknässungseigenschaften der bei den gängigen Tracheal-Kompressen verwendeten Nonwoven-Vliesstoffen erwähnt. Ziel dieser Art Kompressen muss auch sein, dass die Haut des Benutzers trocken bleibt. Die hier in Rede stehenden Nonwoven-Vliesstoffe verfügen nicht über eine so hohe Druckstabilität, dass bei Druckeinwirkung keine Flüssigkeit aus den Kompressen austräte und auf die Körperhaut abgegeben würde, es also zu einer sogenannten Rücknässung kommt. Dieser Zustand wird durch die weiter oben schon beschriebenen Stanzwunden im Kompressenleib und an dem peripheren Rand der Kompressen noch beschleunigt und verstärkt.

Die marktüblichen Tracheal-Kompressen aus ihren, zuvor näher beschriebenen Nonwoven-Vliesstoffen sind wegen der aufwendigen Rohstoffe teuer und hinsichtlich ihres Einsatzes und der dabei gebotenen Sicherheit nur beschränkt tauglich. Ihr Nonwoven-Vliesstoff ist das, was sein Hauptaufgabengebiet, nämlich die Wundversorgung, angeht, monofunktional und monophänotypisch, auf keinen Fall ist er eine Komposition mit kreativer Vielfalt.

Aufgabe der Erfindung ist es nun, die oben genannten Nachteile zu vermeiden und eine Vorrichtung vorzuschlagen, welche in Bezug auf den vollkommen zu vermeidenden Faser- und Faserstaubflug die Vorschriften des MPG (Medizinproduktegesetz) erfüllt und somit am Patienten keinen Schaden anrichtet. Weiterhin ist erwünscht, dass die Vorrichtung kostengünstiger herstellbar und ökologischer ist.

Diese Aufgabe wird bei einer Vorrichtung der eingangs erwähnten und zuvor näher beschriebenen Art dadurch gelöst, dass der Kern absorbierendes Fasermaterial aufweist und die Schnittflächen/Schnittkanten an der Öffnung/am Rand und ggf. an der Durchtrennung abgedeckt, verschlossen und/oder versiegelt sind.

Der Absorptionskern kann aber auch aus den Materialien bestehen, wie bei den bekannten, herkömmlichen Kompressen. Diese sind aber, gemessen an ihrem zugewiesenen Zweck, nämlich dem der Unterfütterung, der Ummantelung, der Lagesicherung der Trachealkanüle und der Feuchtigkeitsabsorption, sehr teuer und ökologisch anfechtbar.

Bei der Herstellung des Kerns absorbierender Produkte im medizinischen und hygienischen Bereich werden heutzutage, aus Gründen der Kosteneinsparung, aber auch wegen der erneuerbaren Resourcen und der hervorragenden Abbaubarkeit, hauptsächlich drei Faserarten eingesetzt: Baumwolle, Viskose und Zellstoff. Die Baumwolle ist zwar die reinste Form der Zellulose und übertrifft die physikalische und chemische Homogenität jeder anderen Pflanzenfaser, scheitert jedoch immer noch oft an dem höheren Preis gegenüber Viskose und besonders gegenüber dem wood pulp - das sind Zellulosefasern, welche aus Holz hergestellt werden

Will man im Zusammenhang mit diesen Materialien ein verfestigtes Vlies erhalten, so wird das sogenannte Airlay-Verfahren, also das Luftlegeverfahren angewendet, an dessen Ende dann schließlich das ,Airlaid'-Vlies, ein integriertes Faservlies, steht. Es wird hierbei die am Beginn des Verfahrens in Rollen vorliegende Zellulose (Fluff) in kurze Fasern geöffnet (Fluff Pulp), im Luftstrom verteilt, auf einem Band abgelegt und schließlich zu einem Airlaid-Vlies verfestigt.

Will man hingegen den Flockenzellstoff (Fluff Pulp) unverfestigt als absorbierenden Kern in ein Hygieneprodukt (disposable) einbringen, so bedient man sich einer Methode, bei welcher zwar die bevorzugt langen Fasern im Luftstrom verteilt, aber nicht auf einem Band abgelegt, sondern von Vakuumpumpen in produktidentische Formgehäuse auf einem Formrad gesaugt werden, so dass am Ende keine Vliesstoffbahnen wie bei dem Airlaid vorliegen, sondern fertige Einweg-Hygiene-Produkte (disposables), wie z.B. Damenbinden, Babywindeln, Inkontinenzvorlagen usw.

Bei dem "Airlaid-Nonwoven" handelt es sich also um ein vielfältig anwendbares Produkt aus dem Ablegeprozess von in der Hauptsache kurzen Fasern im Luftstrom, unter Verwendung verschiedenster Rohstoffe, an erster Stelle Flocken-Zellstoff. Weitere Vorzüge von Airlaid gegenüber den anderen Nonwovens liegen darin, dass es sich nicht nur in unterschiedlichsten Dichten, Dicken, Nass- und Trockenfestigkeiten herstellen lässt, auch die Weichheiten, Flüssigkeitsabsorption und -abgabe, - verteilung, -weitergabe und -speicherung und Saugleistung etc. lassen sich für jede Produktanforderung ideal anpassen.

Das Produktionsverfahren verbindet faser-, granulat- oder flüssige Rohstoffe zu einem optimalen Produkt mit dreidimensionalem Charakter, entweder in einzelnen Bahnen oder homogenen Mischungen. Das Airlaid-Nonwoven zeigt eine große Flexibilität im Eigenschaftsprofil. Es leistet mit seinem Einsatz bei der erfindungsgemäßen Vorrichtung geradezu optimal Hilfe in puncto Beseitigung der Nachteile und Schwächen bei den gängigen Produkten dieser Art auf dem Markt.

Die erfindungsgemäße Vorrichtung stellt bei teilweiser Substitution der teureren traditionellen Vliesstoff-Rohmaterialien durch den deutlich kostengünstigeren Flocken-Zellstoff, bei vermindertem Materialeinsatz, verbessertem Komfort, erhöhtem Saugvolumen, vorzeigbarer Nass- und Trockenfestigkeit und Wirtschaftlichkeit ein rundum in sich verschlossenes, somit faserflug- und schnittstaubfreies System dar, das die Tracheal-Kanüle unter Trockenhaltung der Haut ebenso zuverlässig lagert wie sicher ummantelt. Darüber hinaus ist es in geschlitzter Ausführung leicht auswechselbar, auch bei liegender Kanüle.

Die absorbierende Kernschicht der Erfindung kann einlagig sein und besteht wegen der hohen Saugfähigkeit und wegen des großen Wettbewerbsvorteils als bedeutendem Rohmaterial aus vorzugsweise kurzfaserigem Zellstoff, dem klassischen Rohmaterial der sogenannten Airlay-Technologie. Im Falle eines Airlaids ist die Kernschicht ein verfestigter Vlies. Jedoch lassen sich auch unverfestigte Kernschichten im Rahmen der Erfindung verwirklichen. Dabei handelt es sich dann um einen flockig-daunenhafter Absorptionskern.

Gemäß einer weiteren Ausgestaltung der Erfindung weist der absorbierende Kern Viskose, Baumwolle, Zellstoff und/oder Bi-Komponentenfasern daraus auf. Auf diese Weise wird eine besonders gute Saugfähigkeit erreicht.

Eine weitere Lehre der Erfindung sieht vor, dass die Vorrichtung ein aus einem girlandenartigen Blockmaterial in einer Produktionslinie hergestelltes Multi-Layer-Airlaid-Produkt, also ein mehrschichtiges Erzeugnis, mit mehrschichtigen, supersaugfähigen Kernmaterialien und -schichten, in denen jeder Schicht eine konkrete Aufgabe zukommt, so dass Aufnahme-, Verteilungs-, Flüssigkeitstransport- und Speicherschichten entstehen.

Gemäß einer weiteren Lehre der Erfindung ist der absorbierende Kern mehrschichtig ausgebildet, wobei die untere Kernschicht die Deckschicht und die obere bzw. oberste Kernschicht die Oberflächenschicht trägt. Ohne Zusatz von Klebern und Bindemitteln sind die einzelnen Kernschichten vorzugsweise durch punkt-, strich- und/oder flächenförmige Verbindungen zusammengehalten. Hierbei kann es sich um ein klassisches Vernähen, jedoch auch um eine Verschweißung handeln.

Die Vorrichtung kann auch dadurch charakterisiert sein, dass der Absorptionskern mit einem sogenannten Superabsorber, entweder einen aus neutraler, vernetzter Polyacrylsäure (PANV) oder einem Gel aus nachwachsenden Rohstoffen (Zellulose, Stärke, Pektine), ausgestattet ist, so dass noch mehr Sekret aufgenommen und somit die Vorrichtung entsprechend länger getragen werden kann.

Eine längere Tragedauer kann auch dadurch erreicht werden, dass bei der erfindungsgemäßen Vorrichtung die Deckschicht nach einer weiteren Lehre der Erfindung hydrophile Eigenschaften aufweist. Auch kann die Oberflächenschicht semihydrophob/hydrophil ausgebildet sein. Zum Schutz der Kleidung des Anwenders kann die Oberflächenschicht darüber hinaus auch mit einer vorgelagerten Folie versehen sein. Hierbei kann es sich um eine synthetische Folie handeln, welche gegebenenfalls auch perforiert ausgeführt sein kann.

Eine weitere Ausführung der erfindungsgemäßen Vorrichtung sieht vor, dass die Abdeckung der Absorptionslage sowohl eine zweiteilige als auch eine einteilige Umhüllung sein kann. Beide Male stellt sie vorzugsweise einen Nonwoven-Vliesstoff aus der Gruppe der synthetischen Fasern Polypropylen, Polyesther, Polyethylen, Polyamid, Polyolefin, Copolymer usw. dar, wobei die Deckschicht hydrophil und die Oberflächenschicht wahlweise hydrophob, semihydrophob oder auch hydrophil ausgebildet sein kann. Die körperferne, der Kleidung zugewandte Oberflächenschicht kann anstelle eines Nonwoven-Vlieses aber auch eine fakultativ perforierte synthetische Folie sein, so dass einerseits das Sekret, welches aus der Trachealkanüle austritt und auf die Vorrichtung rinnt in den Absorptionskern aufgenommen werden kann, andererseits aber auch die Kleidung unbeschmutzt und trocken bleibt. In dem Falle, dass die Oberflächenschicht ein Nonwoven-Vlies darstellt, kann diesem zu einem betonteren Kleidungsschutz noch eine vorzugsweise perforierte Folie vorgelagert sein.

Die körpernahe Deckchicht kann wegen der antimikrobiellen Kraft gemäß einer weiteren Ausgestaltung der Erfinduan auch metallbedampft sein. Hier bietet sich beispielsweise eine Aluminium- oder Silberbedampfung an.

Die Vorrichtung der eingangs erwähnten Art kann auch eine antiseptische, antimykotische und/oder fungizide Ausrüstung aufweisen. Die erfindungsgemäße Vorrichtung lässt sich problemlos sterilisieren und, dies ist aus ökologischen Gesichtspunkten besonders wichtig, ist zu großem Teil verrottbar.

Eine weitere Ausgestaltung der Erfindung sieht vor, dass die Fläche der Vorrichtung durch nahtartige Abtrennungen in einzelne Saugfelder unterteilt ist. Diese Nähte können punktuell oder strichförmig ausgeführt sein, wobei in den einzelnen Saugfeldern Sekret und der Schleim in einzelne Kammern verteilt und aufgenommen werden können, wodurch eine gleichmäßige Füllung und Schwereverteilung der gesamten erfindungsgemäßen Vorrichtung ermöglicht wird. Auch dies führt zu einer verlängerten Tragzeit der erfindungsgemäßen Vorrichtung.

Eine weitere Lehre der Erfindung sieht vor, dass sich von der Öffnung bis zum äußeren Rand eine Durchtrennung, also ein Schlitz, erstreckt. Auf diese Weise ist es möglich, die Vorrichtung auch um ein in situ befindliches Kanülenrohr herum anzulegen. Gemäß einer weiteren Ausbildung der Erfindung verläuft die Durchtrennung so, dass die voneinander getrennten, gegenüberliegenden Bereiche lösbar in formschlüssiger Verbindung stehen. Dabei kann die Verbindung schwalbenschwanz-, pilzartig sein oder einhakende oder ähnlich ineinandergreifende Elemente aufweisen.

Es ist klar, dass bei der Ausführung mit Durchtrennung auch die Schnittflächen der Durchtrennung dicht abgedeckt, verschlossen und/oder versiegelt sein müssen, um einerseits keine Fasern oder Faserstäube freizusetzen oder andererseits das aufgenommene Sekret in der Vorrichtung zu halten. Dabei kann die Abdeckung, Verschließung, Versiegelung an der Öffnung, am Rand und/oder an der Durchtrennung mittels Hochfrequenz oder durch entsprechende thermoplastische oder mechanische Behandlung erfolgen.

Gemäß einer weiteren Ausbildung der Erfindung sind die Schnittflächen der Durchtrennung so abgedeckt, verschlossen und/oder versiegelt, dass die Gesamtstärke der einzelnen Schichten nicht oder nur minimal verringert ist. Auf diese Weise wird einerseits die formschlüssige Verbindung der beiden benachbarten Vorrichtungsteile und andererseits auch deren Saugfähigkeit nicht beeinträchtigt.

Schließlich sieht eine weitere Lehre der Erfindung vor, dass die beiden Seiten im Bereich der Durchtrennung vor dem erstmaligen Gebrauch mit dünnen Stegen verbunden sind. Diese bevorzugte Ausführungsform hat den Vorteil, dass nach dem Stanzen der Durchtrennung eine bessere Handhabung der Vorrichtung für den Transport und die Lagerung möglich ist, da die beiden später zu trennenden Bereiche mittels der Stege noch in Verbindung stehen. Es handelt sich also gewissermaßen um eine Perforation, welche erst beim endgültigen Anlegen der Vorrichtung durch Zerstören der Stege geöffnet werden muss.

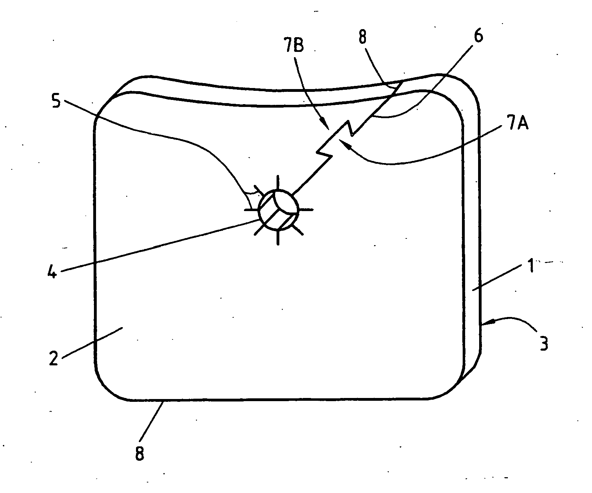

Die Erfindung wird nachfolgend anhand einer lediglich bevorzugtes Ausführungsbeispiel darstellenden Zeichnung näher erläutert. Die einzige Figur zeigt ein Ausführungsbeispiel einer erfindungsgemäßen Vorrichtung in perspektivischer Ansicht. Die Erfindung ist jedoch nicht auf eine solche Ausführung beschränkt.

Die dargestellte Trachealkompresse weist zunächst einen als Saugkompresse ausgebildeten Kern 1 mit einer zu der Kleidung weisenden Oberflächenschicht 2 und einer dem Körper zugewandten Deckschicht 3 auf. Im dargestellten Ausführungsbeispiel mittig befindet sich im oberen Drittel eine Öffnung 4 zur Aufnahme einer nicht dargestellten Trachealkanüle. Über den Umfang der Öffnung 4 sternförmig verteilt sind Querstanzungen 5 erkennbar, die ein Aufweiten der Öffnung 4 auf unterschiedliche Durchmesser der jeweiligen Kanülenrohre ermöglichen.

Damit nun die erfindungsgemäße Vorrichtung auch bei liegender Kanüle gewechselt werden kann, ist in der einzigen Figur eine Durchtrennung 6 vorgesehen, welche erfindungsgemäß bevorzugt schwalbenschwanzartig von der Öffnung 4 zum oberen Rand 8 der Vorrichtung verläuft, um ein formschlüssiges Ineinandergreifen der von der Durchtrennung 6 getrennten, gegenüberliegenden Bereiche 7A und 7B der Trachealkompresse zuverlässig formschlüssig miteinander zu verbinden. Diese Bereiche können, wie erwähnt jedoch nicht dargestellt, nach der Herstellung mit dünnen Stegen verbunden sein, die beim erstmaligen Öffnen der Durchtrennung zerstört werden. Es ist schnell ersichtlich, dass ein leichtes Verschieben der Bereiche 7A bzw. 7B der Kompresse aus ihrer flächigen Ebene heraus ein Öffnen der Durchtrennung 6 erlaubt, um die Trachealkompresse anlegen zu können, ohne dass die Trachealkanüle eine Lageänderung erfährt.

Es ist erkennbar, dass die erfindungsgemäße Vorrichtung auch nach längerem Tragen aufgrund ihres Eigengewichtes zuverlässig in ihrer Lage verbleibt, ohne dass sich die Kompresse selbständig durch Öffnen der Durchtrennung 6 vom Rohr der Kanüle lösen kann.

## Patentansprüche

1. Vorrichtung zur Unterfütterung, Ummantelung und Lagesicherung von Trachealkanülen mit einer dem Körper zugewandten Deckschicht (3), einer zu der Kleidung weisenden Oberflächenschicht (2) und einem zwischen Deckschicht (3) und Oberflächenschicht (2) befindlichen ein- oder mehrschichtigen Kern (1), mit einer alle Schichten (3, 1, 2) durchbrechenden, im wesentlichen runden Öffnung (4) zur Aufnahme der Trachealkanüle, wobei zwischen der Öffnung (4) und dem äußeren Rand (8) eine Durchtrennung (6) vorgesehen sein kann
**dadurch gekennzeichnet, dass**
der Kern (1) absorbierendes Fasermaterial aufweist und die Schnittflächen/Schnittkanten an der Öffnung (4) und ggf. an der Durchtrennung (6) und am Rand (8) abgedeckt, verschlossen und/oder versiegelt sind.

2. Vorrichtung nach Anspruch 1,
**dadurch gekennzeichnet, dass**
die absorbierende/n Kernschicht/en (1) Viskose, Baumwolle und/oder Zellstoff aufweist/aufweisen.

3. Vorrichtung nach Anspruch 1 oder 2,
**dadurch gekennzeichnet, dass**
die absorbierende/n Kernschicht/en (1) Bi-Komponentenfasern aufweist/aufweisen.

4. Vorrichtung nach einem der Ansprüche 1 bis 3,
**dadurch gekennzeichnet, dass**
die absorbierende/n Kernschicht/en (1) verfestigt ist/sind.

5. Vorrichtung nach einem der Ansprüche 1 bis 3,
**dadurch gekennzeichnet, dass**
die absorbierende/n Kernschicht/en (1) unverfestigt ist/sind.

6. Vorrichtung nach einem der Ansprüche 1 bis 5
**dadurch gekennzeichnet, dass**
der absorbierende Kern (1) mehrschichtig ist und dass die untere Kernschicht die Deckschicht (3) und die ober(st)e Kernschicht die Oberflächenschicht (2) trägt.

7. Vorrichtung nach einem der Anspruch 6,
**dadurch gekennzeichnet, dass**
die einzelnen Kernschichten durch punkt-, strich- und/oder flächenförmige Verbindungen zusammengehalten werden.

8. Vorrichtung nach einem der Ansprüche 1 bis 7,
**dadurch gekennzeichnet, dass**
der absorbierende Kern (1) eine superabsorbierende Substanz aufweist.

9. Vorrichtung nach einem der Ansprüche 1 bis 8,
**dadurch gekennzeichnet, dass**
die Deckschicht (3) hydrophile Eigenschaften aufweist.

10. Vorrichtung nach einem der Ansprüche 1 bis 9,
**dadurch gekennzeichnet, dass**
die Oberflächenschicht (2) semihydrophob/hydrophil ausgebildet ist.

11. Vorrichtung nach einem der Ansprüche 1 bis 10,
**dadurch gekennzeichnet, dass**
der Oberflächenschicht eine Folie vorgelagert ist.

12. Vorrichtung nach Anspruch 11,
**dadurch gekennzeichnet, dass**
die Folie eine, ggf. perforierte, synthetische Folie ist.

13. Vorrichtung nach einem der Ansprüche 1 bis 12,
**dadurch gekennzeichnet, dass**
Deckschicht (3) und/oder Oberflächenschicht (2) aus Nonwoven-Vliesstoffen bestehen.

14. Vorrichtung nach einem der Ansprüche 1 bis 13,
**dadurch gekennzeichnet, dass**
die Deckschicht (3) metallbedampft ist.

15. Vorrichtung nach einem der Ansprüche 1 bis 14,
**gekennzeichnet durch**
eine antiseptische, antimykotische und/oder fungizide Ausführung.

16. Vorrichtung nach einem der Ansprüche 1 bis 15,
**dadurch gekennzeichnet, dass**
die Fläche der Vorrichtung durch nahtartige Abtrennungen in einzelne Saugfelder unterteilt ist.

17. Vorrichtung nach einem der Ansprüche 1 bis 16, wobei sich von der Öffnung (4) bis zum äußeren Rand (8) eine Durchtrennung (6) erstreckt,
**dadurch gekennzeichnet, dass**
die Durchtrennung (6) so verläuft, dass die voneinander getrennten, gegenüber liegenden Bereiche (7A, 7B) lösbar in formschlüssiger Verbindung stehen.

18. Vorrichtung nach Anspruch 17,
**dadurch gekennzeichnet, dass**
die Verbindung schwalbenschwanz- oder pilzartig ineinandergreifende Elemente aufweist.

19. Vorrichtung nach Anspruch 17 oder 18,
**dadurch gekennzeichnet, dass**
die beiden Seiten im Bereich der Durchtrennung (6) vor dem erstmaligen Gebrauch mit dünnen Stegen verbunden sind.

20. Vorrichtung nach einem der Ansprüche 17 bis 19,
**dadurch gekennzeichnet, dass**
die Schnittflächen der Durchtrennung (6) so abgedeckt, verschlossen und/oder versiegelt sind, dass die Gesamtstärke der einzelnen Schichten (2, 1, 3) nicht oder nur minimal verringert ist.

## Claims

1. Device for the underpadding, cladding, and position securing of tracheal cannulae, with a cover layer (3) facing the body, a surface layer (2) facing the clothing and a single-layer or multi-layer core (1) located between cover layer (3) and surface layer (2), with an essentially round opening (4) penetrating through all the layers (3, 1, 2) to accommodate the tracheal cannula, wherein a continuous separation (6) can be provided between the opening (4) and the outer edge (8), **characterised in that** the core (1) has absorbent fibre material and the cut surfaces/cut edges are covered, closed, and/or sealed at the opening (4) and if required at the continuous separation (6) and at the edge (8).

2. Device according to Claim 1, **characterised in that** the absorbent core layer(s) (1) has/have viscose, cotton, and/or cellulose.

3. Device according to Claim 1 or 2, **characterised in that** the absorbent core layer(s) (1) has/have bicomponent fibres.

4. Device according to any one of Claims 1 to 3, **characterised in that** the absorbent core layer(s) (1) is/are secured.

5. Device according to any one of Claims 1 to 3, **characterised in that** the absorbent core layer(s) (1) is/are unsecured.

6. Device according to any one of Claims 1 to 5, **characterised in that** the absorbent core (1) is multi-layered, and **in that** the lower core layer carries the cover layer (3) and the upper(most) core layer carries the surface layer (2).

7. Device according to Claim 6, **characterised in that** the individual core layers are held together by point connections, line connections, and/or surface-form connections.

8. Device according to any one of Claims 1 to 7, **characterised in that** the absorbent core (1) has a superabsorbent substance.

9. Device according to any one of Claims 1 to 8, **characterised in that** the cover layer (3) has hydrophilic properties.

10. Device according to any one of Claims 1 to 9, **characterised in that** the surface layer (2) is designed as semi-hydrophobic/hydrophilic.

11. Device according to any one of Claims 1 to 10, **characterised in that** a film is located in front of the surface layer.

12. Device according to Claim 11, **characterised in that** the film is a synthetic film, perforated if appropriate.

13. Device according to any one of Claims 1 to 12, **characterised in that** the cover layer (3) and/or the surface layer (2) consist of non-woven fleece materials.

14. Device according to any one of Claims 1 to 13, **characterised in that** the cover layer (3) is of vapour-deposited metal.

15. Device according to any one of Claims 1 to 14, **characterised by** an antiseptic, antimycotic, and/or fungicidal implementation.

16. Device according to any one of Claims 1 to 15, **characterised in that** the surface of the device is subdivided by seam-like partitions into individual suction fields.

17. Device according to any one of Claims 1 to 16, wherein a continuous separation (6) extends from the opening (4) as far as the outer edge (8), **characterised in that** the continuous separation (6) runs in such a way that the areas (7A, 7B) separated from one another and located opposite one another are in positive connection in a detachable manner.

18. Device according to Claim 17, **characterised in that** the connection has mutually-engaging dovetail or mushroom-shaped elements.

19. Device according to Claim 17 or 18, **characterised in that** the two sides are connected in the area of the continuous separation (6) by thin webs before use for the first time.

20. Device according to any one of Claims 17 to 19, **characterised in that** the cut surfaces of the continuous separation (6) are covered, closed, and/or sealed in such a way that the overall thickness of the individual layers (2, 1, 3) is not reduced, or only minimally.

## Revendications

1. Dispositif pour soutenir, envelopper et immobiliser des canules trachéales, qui présente une couche de recouvrement (3) tournée vers le corps, une couche de surface (2) tournée vers les vêtements et une âme (1) en une ou plusieurs couches située entre la couche de recouvrement (3) et la couche de surface (2) ainsi qu'une ouverture (4) essentiellement circulaire, qui traverse toutes les couches (3, 1, 2) et qui reprend la canule trachéale, une séparation (6) pouvant être prévue entre l'ouverture (4) et le bord extérieur (8),
**caractérisé en ce que**
l'âme (1) présente un matériau fibreux absorbant et les surfaces de coupe ou les bords de coupe de l'ouverture (4) et éventuellement de la séparation (6) et du bord (8) sont recouverts, fermés et/ou scellés.

2. Dispositif selon la revendication 1, **caractérisé en ce que** la ou les couches absorbantes de l'âme (1) présentent de la viscose, du coton et/ de la cellulose.

3. Dispositif selon les revendications 1 ou 2, **caractérisé en ce que** la ou les couches absorbantes de l'âme (1) présentent des fibres à deux composants.

4. Dispositif selon l'une des revendications 1 à 3, **caractérisé en ce que** la ou les couches absorbantes de l'âme (1) sont solidifiées.

5. Dispositif selon l'une des revendications 1 à 3, **caractérisé en ce que** la ou les couches absorbantes de l'âme (1) ne sont pas solidifiées.

6. Dispositif selon l'une des revendications 1 à 5, **caractérisé en ce que** l'âme absorbante (1) présente plusieurs couches et **en ce que** la couche inférieure d'âme porte la couche de recouvrement (3) et la couche supérieure d'âme porte la couche de surface (2).

7. Dispositif selon la revendication 6, **caractérisé en ce que** les différentes couches d'âme sont maintenues ensemble par des liaisons ponctuelles, linéaires ou de surface.

8. Dispositif selon l'une des revendications 1 à 7, **caractérisé en ce que** l'âme absorbante (1) présente une substance superabsorbante.

9. Dispositif selon l'une des revendications 1 à 8, **caractérisé en ce que** la couche de recouvrement (3) possède des propriétés hydrophiles.

10. Dispositif selon l'une des revendications 1 à 9, **caractérisé en ce que** la couche de surface (2) est semi-hydrophobe ou hydrophile.

11. Dispositif selon l'une des revendications 1 à 10, **caractérisé en ce qu'**un film est superposé en avant de la couche de surface.

12. Dispositif selon la revendication 11, **caractérisé en ce que** le film est un film synthétique éventuellement perforé.

13. Dispositif selon l'une des revendications 1 à 12, **caractérisé en ce que** la couche de recouvrement (3) et/ou la couche de surface (2) sont constituées de feutres non tissés.

14. Dispositif selon l'une des revendications 1 à 13, **caractérisé en ce que** la couche de recouvrement (3) est formée par dépôt de vapeur métallique.

15. Dispositif selon l'une des revendications 1 à 14, **caractérisé en ce qu'**il est rendu antiseptique, antimycotique et/ou fongicide.

16. Dispositif selon l'une des revendications 1 à 15, **caractérisé en ce que** la surface du dispositif est divisée en différentes zones d'aspiration par des séparations de type cousu.

17. Dispositif selon l'une des revendications 1 à 16, dans lequel une séparation (6) s'étend entre l'ouverture (4) et le bord extérieur (8), **caractérisé en ce que** la séparation (6) s'étend de telle sorte que des zones (7A, 7B) mutuellement séparées et situées l'une en face de l'autre sont reliées de manière libérable en correspondance géométrique.

18. Dispositif selon la revendication 17, **caractérisé en ce que** la liaison présente des éléments en queue d'aronde ou en champignon qui s'engagent les uns dans les autres.

19. Dispositif selon les revendications 17 ou 18, **caractérisé en ce qu'**avant sa première utilisation, les deux côtés sont reliés par de minces traverses dans la zone de la séparation (6).

20. Dispositif selon l'une des revendications 17 à 19, **caractérisé en ce que** les surfaces de coupe de la séparation (6) sont recouvertes, fermées et/ou scellées de telle sorte que l'épaisseur totale des différentes couches (2, 1, 3) ne soit pas diminuée ou ne le soit que de manière minimale.
